# EUROPEAN PATENT APPLICATION

(11) **EP 1 535 617 A1**
(43) Date of publication of application: **01.06.2005**
(21) Application number: 03738636.4
(22) Date of filing: 02.07.2003
(51) Int. Cl.: A61K 31/5575, A61K 31/196, A61K 9/08, A61P 27/02, A61P 29/00

(54) **REMEDY OR PREVENTIVE FOR KERATOCONJUNCTIVAL EPITHELIAL CELL INJURY**

(30) Priority: 02.07.2002 JP 2002193603
(71) Applicant: Wakamoto Pharmaceutical Co., Ltd., Chuo-ku, Tokyo 103-8330 (JP)
(72) Inventor: AKIBA, Kiyoshi c/o Wakamoto Pharmaceutical Co. Ltd, Chuo-Ku, Toky o 103-8330 (JP); HOSHINA, Futoshi c/o Wakamoto Pharmaceutical Co.Lt, Chuo-Ku, Tokyo 103-8330 (JP); AGATA, Mitsuzi c/o Wakamoto Pharmaceutical Co. Ltd, Chuo-Ku, Tokyo 103-8330 (JP)
(74) Representative: VOSSIUS & PARTNER
(86) International application number: PCT/JP2003/008409
(87) International publication number: WO 2004/004738

(57) **Abstract**

The present invention provides an eye drop for treating or preventing disorders of corneal and/or conjunctival epithelial cells comprising prostaglandin E's as effective components; and an eye drop for treating or preventing disorders of corneal and/or conjunctival epithelial cells comprising prostaglandin E's and an anti-inflammatory agent as effective components. The eye drop shows a high effect of treating and/or preventing disorders of cornea or conjunctiva originated from physical, chemical and/or mechanical damages of the cornea or conjunctiva as well as endogenous disorders of cornea or conjunctiva.

## Description

### Technical Field

The present invention relates to an ophthalmologic drug for treating or preventing disorders of corneal and/or conjunctival epithelial cells. More particularly, the present invention pertains to an eye drop having an effect of treating and/or preventing disorders of corneal and/or conjunctival epithelial cells caused due to the use of a drug, in particular, a medicine such as an anti-inflammatory agent and disorders of the foregoing epithelial cells caused due to the reduction of, for instance, prostaglandins.

### Background Art

The cornea is a tissue existing on the anterior pole of the eyeball, covering the anterior chamber of the lens, while maintaining the transparency of the lens and having a well-ordered layer structure which comprises a corneal epithelium, a Bowman membrane, a keratocyte layer, a Descemet membrane and a corneal endothelium. Disorders of the corneal and/or conjunctival epithelial cells include those accompanied by various endogenic ophthalmologic diseases such as herpes corneae, bacterial helcoma (corneal ulcer), neuroparalytic keratitis, diabetic keratitis, Sjogrens syndromes (diseases) and dry eyes; exogenous diseases caused by, for instance, ophthalmologic operations, the use of drugs, trauma and the applications of contact lenses; or those originated from physical or chemical damages and these disorders may be advanced to inveterate corneal disorders in which any epithelium cannot be regenerated, depending on the causes of these diseases and result in the impaired vision and/or the loss of eyesight (ablepsia) in the worst cases. When the corneal epithelial cells are damaged for some reason in the course of the formation of the regular and well-ordered lattice-like structure of the corneal keratocyte layer, it would be assumed that a substance capable of serving as the corneal cells plays an important role in the healing of the damaged cornea.

Keratitis such as helcoma (corneal ulcer) is caused as a disease complicated with, for instance, the operation of cataract or retina, the removal of vitreous body and the radiotherapy of corneal cancers. In this connection, steroids such as betamethasone and non-steroidal anti-inflammatory drugs such as diclofenac sodium have been used in the treatment and prevention of such keratitis. These drugs are quite effective ones for the treatment of inflammatory symptoms, but it has been known that the use of these drugs, in particular, over a long period of time would induce disorders of corneal and/or conjunctival epithelial cells and corneal and/or conjunctival ulcers and led to the synchysis of cornea and even to the loss of eyesight (Julianne C. Lin et al., ARCH. OPHTHALMOL., 118, AUG. 2000, pp. 1129-1132).

To recover the eyesight, it would be necessary that the normal regeneration and coverage of the corneal epithelium must immediately be taken place. The healing mechanism of disorders of corneal and/or conjunctival epithelial cells includes first to third phases, in which the first phase comprises the adhesion, extension and/or migration of epithelial cells; the second phase comprises the proliferation or growth of epithelial cells; and the third phase comprises the differentiation of the epithelial cells. The damaged epithelial cells are not regenerated into the normal epithelium having a well-ordered layer structure only when the damaged epithelial cells pass through these three phases. Accordingly, there has been desired for the development of a drug capable of restoring these processes. In addition, there has also been known, for instance, eye drops containing hyaluronic acid and fibronectin, but they are not satisfied with their effect and, for this reason, there has also been desired for the development of a drug having a satisfactory drug efficacy.

On the other hand, prostaglandins (hereunder simply referred to as "PG") are included in the tissues and organs of mammals, they constitute a group of organic carboxylic acids having various physiological activities and have prostanoic acid skeletons as general structural characteristics.

It has been recognized that PGE₁, PGE₂ and PGE₃ have various functions such as a vasodilation effect, an anti-hypertensive effect, an effect of reducing the acid secreted in the stomach, an effect of promoting or exasperating the motions of intestines, an effect of uterine contraction, a diuretic effect, a bronchodilative effect and an anti-ulcerative effect. Regarding the application of PG in the field of ophthalmology, it has been known that homologues of PGF_{2α} show an intraocular pressure-reducing effect (Alm., The Potential of Prostaglandin Derivatives in Glaucoma Therapy, Current Opinion in Ophthalmology, Vol. 4, No. 11, pp. 44-50, 1993).

Pedro Cortina et al. (Graefe's Arch. Clin. Exp. Ophthalmol., 1997, 235:180-185) state that, in the in vitro-cultivation of epithelial cells of lenses, which are currently anaerobic and in reduced states, the after-cataract as a side-effect observed after the cataract operation can be inhibited by the addition of 25, 50 and 100 µ M of diclofenac sodium to the culture medium, through the control of the proliferation of the epithelial cells. Further the authors of this article suggest that PGE₂ would be involved in the pathema due to cell proliferation such as the after-cataract since the addition of 1, 10, 50 and 100 µ M of PGE₂ to the culture medium permits the growth of the epithelial cells. However, this article does not disclose any treatment or alleviation of the disorders of corneal epithelial cells at all.

YAMAMOTO reports, in the Bulletin of Japan Ophthalmologic Society (103 (3), p. 164, 1999), the results obtained in the studies of rabbit's corneal pieces, which are as follows: A prostaglandin production-inhibitory agent such as diclofenac sodium or indometacin alone does not affect the extension of the corneal epithelial cells, but when an epithelium growth factor (EGF) is added to the culture medium simultaneously with the foregoing agent, the latter would inhibit the epithelium extension-promoting effect due to the EGF. In the cultivation of the epithelial cells, agents such as diclofenac sodium do not affect the proliferation of the epithelium and the proliferation-promoting effect of the EGF, but the concentrations of PGE₂ and PGF_{2α} in the solution containing the epithelial cells are increased to a level higher than that observed for the control groups when EGF is added to the culture medium.

TSUKIYAMA et al. as members of the same research group set forth, in the collected resumes for the lectures held by the Ophthalmologic Society in the central districts of Japan (E-W-2; 2000), their opinion to the effect that when, in the monolayer culture of corneal epithelial cells, a defect having a predetermined area is formed, the defected portion is reduced through the addition of EGF and a prostaglandin production-inhibitory agent can significantly inhibit the effect of promoting damaged epithelium-healing action upon the simultaneous addition thereof with EGF, while the former alone cannot affect the damaged area of the epithelium. These authors state that the addition of EGF would significantly increase the concentrations of PGE₂ and PGF_{2α} in the culture medium, but the resume does not include any disclosure concerning the externally added PG.

TSUKIYAMA et al. likewise disclose, in the Bulletin of Japan Ophthalmologic Society (105, p. 35, 2001), as follows: when, in the monolayer culture of corneal epithelial cells, a damaged area is formed, the addition of EGF would promote the healing of the wound on the damaged area, while the addition of diclofenac sodium would inhibit the damaged epithelium-healing. In addition, it has been recognized that the additional incorporation, into the culture medium, of latanoprost as a derivative of PGF _{2α} can recover the healing of the damaged epithelium, but any such effect cannot be obtained by the addition of latanoprost alone. Moreover, a derivative of PGE₂: 16,16-dimethyl prostaglandin E₂ (16,16-dimethyl PGE2) does not show any effect on the healing of the damaged corneal epithelium in the both cases in which the derivative is added alone or simultaneously with diclofenac sodium. In any case, these results do not teach or suggest any effect of the PGE₂ derivative on the disorders of corneal and/or conjunctival epithelial cells at all.

As a typical example of the side-effect of the anti-inflammatory agent, there has been known disorders of gastric mucous membrane observed when the agent is orally administered. The PG's have an effect of protecting the gastric mucous membrane and, for instance, Scand. J. Rheumatol., 1992, 21 (2): 85-91 discloses that a medical mixture containing diclofenac sodium and misoprostol as a derivative of PG (hereunder referred to as "MS") inhibits the occurrence of any gastric ulcer and still maintains its anti-inflammatory activity.

Similarly, WO 92/21350 discloses that the simultaneous use of a non-steroidal anti-inflammatory agent with prostaglandin E's such as MS, prostaglandin E₁ (PGE₁), prostaglandin E₂ (PGE₂), 15R-methyl prostaglandin E₂ (15R-methyl PGE₂), 16,16-dimethyl prostaglandin E1 (16,16-dimethyl PGE₁), 16,16-dimethyl prostaglandin E₂ (16,16-dimethyl PGE₂) may relieve or eliminate the side-effect and that the medical mixture would be effective as an agent for treating arthrorheumatism and osteoporosis.

To alleviate such disorders of gastric mucous membrane, in case of the non-steroidal anti-inflammatory agent, there have been developed drugs having selectivity to cyclooxygenase (hereunder referred to as "COX") or a drug having selectivity to COX-2 such as meloxicam and these drugs are valued to a reasonable extent in the market. However, the disorders of gastric mucous membrane have not yet been eliminated completely. This is because the selectivity of these drugs is rather relative one and the activity of COX-2 still remains to some extent.

In the field of ophthalmology, there has been disclosed such a technique that the damage of corneal cells observed during the cataract operation may be relieved by the use of a COX-2-selective anti-inflammatory agent such as meloxicam in place of a non-selective COX-inhibitor as an anti-inflammatory agent (WO 99/59634). However, the selectivity to COX of these drugs is simply ones relatively estimated and the COX-2 activity of the drugs is not completely controlled. Accordingly, another means should be used for treating or preventing disorders of corneal and/or conjunctival epithelial cells.

### Disclosure of the Invention

Accordingly, it is an object of the present invention to provide a drug for treating or preventing disorders of corneal and/or conjunctival epithelial cells, in particular, the disorders thereof originated from the reduction of the concentrations of PGE's or the disorders thereof caused by the use of drugs, while taking into consideration the foregoing actual circumstances.

The present invention relates to an eye drop for treating or preventing disorders of corneal and/or conjunctival epithelial cells attributable to a variety of factors, which comprises prostaglandins as effective components.

More specifically, the present invention provides an eye drop for treating or preventing disorders of corneal and/or conjunctival epithelial cells detailed below:
1. An eye drop for treating or preventing disorders of corneal and/or conjunctival epithelial cells comprising prostaglandin E's as effective components.
2. The eye drop as set forth in the foregoing item 1, wherein the prostaglandin E's are selected from the group consisting of misoprostol, prostaglandin E1 (PGE1), prostaglandin E2 (PGE2), 15R-methyl prostaglandin E2 (15R-methyl PGE2), 16,16-dimethyl prostaglandin E1 (16,16-dimethyl PGE1), 16,16-dimethyl prostaglandin E2 (16,16-dimethyl PGE2), oxyprostol and lower alkyl esters thereof.
3. The eye drop as set forth in the foregoing item 1 or 2, wherein the disorders of corneal and/or conjunctival epithelial cells are caused due to the reduction of prostaglandin E's.
4. The eye drop as set forth in the foregoing item 1 or 2, wherein the disorders of corneal and/or conjunctival epithelial cells are caused due to the use of a drug.
5. The eye drop as set forth in the foregoing item 4, wherein the causative drug for the disorders of corneal and/or conjunctival epithelial cells is an anti-inflammatory agent.
6. The eye drop as set forth in the foregoing item 5, wherein the anti-inflammatory agent as a causative drug for the disorders of corneal and/or conjunctival epithelial cells is a drug non-selectively inhibiting cyclooxygenase.
7. The eye drop as set forth in the foregoing item 6, wherein the drug non-selectively inhibiting cyclooxygenase as a causative drug for the disorders of corneal and/or conjunctival epithelial cells is diclofenac sodium.
8. An eye drop for treating or preventing disorders of corneal and/or conjunctival epithelial cells comprising prostaglandin E's and a causative drug for the disorders of corneal and/or conjunctival epithelial cells, as effective components.
9. The eye drop as set forth in the foregoing item 8, wherein the causative drug for the disorders of corneal and/or conjunctival epithelial cells is an anti-inflammatory agent.
10. The eye drop as set forth in the foregoing item 9, wherein the ratio, by mass, of the prostaglandin E's to the anti-inflammatory agent ranges from 1:25 to 50000.
11. The eye drop as set forth in the foregoing item 10, wherein the prostaglandin E is misoprostol and the anti-inflammatory agent is diclofenac sodium.
12. The eye drop as set forth in the foregoing item 11, wherein the concentration of the misoprostol ranges from 0.01 µ M to 10 µ M.
13. The eye drop as set forth in the foregoing item 11, wherein the concentration of the diclofenac sodium ranges from 0.01% to 0.5%.

### Brief Description of the Drawings

Fig. 1 is a graph showing the effect of MS according to the present invention on disorders of corneal epithelial cells.
Fig. 2 is a graph showing the effect of MS according to the present invention on disorders of conjunctival epithelial cells.

### Best Mode for Carrying Out the Invention

The prostaglandins usable in the present invention may be any one insofar as it belongs to the prostaglandin E's, but preferably used herein include, for instance, MS, PGE₁, PGE₂, 15R-methyl PGE₂, 16,16-dimethyl PGE₁, 16,16-dimethyl PGE₂, oxyprostol and lower alkyl esters thereof (such as lower alkyl esters having 1 to 5 carbon atoms), with MS being most preferably used herein.

In the present invention, MS may be used alone, but when using a causative drug for disorders of corneal and/or conjunctival epithelial cells such as a non-selective anti-inflammatory agent, it may likewise be formulated into a medical mixture with such a drug.

The non-selective anti-inflammatory agents usable in the present invention are, for instance, diclofenac, indometacin, flurbiprofen and ibuprofen, with diclofenac being preferably used herein.

The amount of the prostaglandins to be used desirably ranges from 0.001 to 10 µ M for the both eye drops simply comprising the same and comprising the same in the form of a medical mixture, but the amount may be not less than 10 µ M, while taking into consideration the dilution of the eye drop with the lacrima. Moreover, when they are used in combination with an anti-inflammatory agent such as diclofenac sodium, the ratio (by mass) of the amount of the anti-inflammatory agent to that of the MS to be used desirably ranges from 1:25 to 50000 although the ratio may vary depending on the concentration of the anti-inflammatory agent. When using an anti-inflammatory agent in an eye drop, it is in general used in an amount ranging from 0.01 to 0.5% and most preferably 0.1%. In such a case, the ratio (by mass) of the amount of the anti-inflammatory agent to that of the MS to be used desirably ranges from 1:25 to 50000. However, the concentrations of these components are not limited to the foregoing range in the present invention.

The term "eye drop" used herein includes all the dosage forms used for the treatment of eyes such as eye solutions, ophthalmic circulation liquids (perfusions), ophthalmic ointments or freeze-dried drugs. The eye drop of the present invention may be used as a drug for treating or preventing disorders of corneal and/or conjunctival epithelial cells in any dosage form, but it is particularly preferred to use the same in the form of an eye solution, an ophthalmic perfusion, a cream, an ophthalmic ointment or a freeze-dried drug. However, the present invention is not restricted to these dosage forms. When the dosage form selected is an eye solution, an ophthalmic ointment or a cream, they may comprise conentionally used additives, for instance, preservatives such as sodium dehydroacetate and methyl para-oxy-benzoate; an isotonicity such as sodium chloride and glycerin; a thickener such as carboxy methyl cellulose and polyvinyl alcohol; and a stabilizer such as polysaccharides and sodium edetate. However, the additives are not restricted to these specific ones and any additives physiologically acceptable may be used without any restriction. Buffer solutions to be incorporated into the foregoing various kinds of dosage forms are preferably those having isotonicity, free of any stimulus and having a buffering pH range of from 4 to 9 or from 5 to 9.

The following are examples of eye drops according to the present invention.

### Preparation Example 1

| | |
|---|---|
| Misoprostol | 0.002% |
| Phosphate buffer solution | 1.0% |
| Benzalkonium chloride | 0.001% |
| Polysolvate 80 | 0.5% |
| Pure water | Ad. to 100% |

The method of using the drug for treating a disease of cornea according to the present invention and the dose thereof may vary depending on various factors such as the symptom and age of each particular patient, but an eye drop is commonly and desirably administered 1 to 5 times a day in an amount of 1 to 5 drops per dose in case of an eye solution. On the other hand, in case of an ophthalmic ointment, it is in general applied to the interior of the corneal and/or conjunctival sac in a sufficient amount, 1 to 3 times a day.

The present invention will hereunder be described in more detail with reference to the following Examples, but the present invention is not restricted to these specific Examples.

### Examples

### [Test Example]: Effect of MS on Disorders of Human Corneal Epithelial Cells and Human Conjunctival Epithelial Cells Caused Due to Use of Diclofenac Sodium

Cells Used: The corneal cell used herein was SV40 immortalized human corneal epithelial cell strain (HCEC) (Araki-Sasaki et al., IOVS, 1995, 36:614-621) and the conjunctival cell used herein was Chang's human conjunctival epithelial cell strain (CCL-20.2) (ATC CCCL-20.2).
Experiments: The cells were cultivated to a density of 5x10⁴ cells/mL in a growth medium containing 10% serum, inoculated in a 96-well multiplate in an amount of 100 µ L/well and cultivated at 37°C overnight. After the cultivation thereof to a confluent of 30 to 50%, the culture medium was dissolved in a fresh culture medium, followed by the addition of stepwise diluted MS and further diclofenac sodium and the subsequent cultivation at 37°C for 24 hours. After the cultivation, each well was washed, followed by the addition of a fresh culture medium (100 µ L/well) and WST-8 (10 µ L/well; Cell Counting Kit-8 available from DOJIN Chemical Co., Ltd.), subsequent cultivation for 1.5 hour and the determination of the absorbance at 450-660 nm to thus calculate the viable cell count.
Method for Evaluating Toxicity: A test group free of any candidate compound was used as a negative control, the absorbance thereof was assumed to be 100% and a dose-correlation curve was prepared, which was expressed as the cell survival rates as a function of the concentration of each test substance.

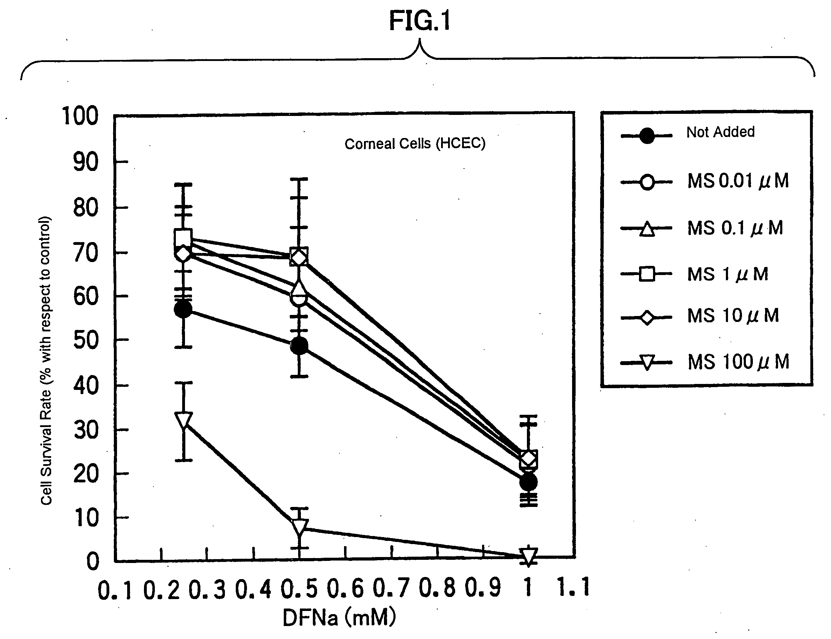

The following Table 1 and Fig. 1 show the effect of Misoprostol to protect SV40 immortalized human corneal epithelial cells from corneal disorders caused due to the action of a non-steroidal anti-inflammatory agent.

Diclofenac sodium as a non-steroidal anti-inflammatory agent may damage the cells at a concentration ranging from 0.25 to 1 mM and misoprostol alleviated the disorders of the cells in an amount ranging from 0.01 to 100 µ M. In this connection, misoprostol per se caused disorders of cells at a high concentration on the order of 100 µ M.

**Table 1:**

| Effect of Misoprostol on Disorders of Corneal Epithelial Cells due to Diclofenac Sodium | | | | | | | |
|---|---|---|---|---|---|---|---|
| Concn. of MS (µ M) | Concentration of Diclofenac Sodium (mM) | | | | | | |
| | 0 | 0.25 | p Value | 0.5 | p Value | 1 | p Value |
| 0 | 100(%) | 56.9±8.7 | | 48.2±6.7 | | 17.3±5.4 | |
| 0.01 | 87.2±13.2 | 69.9±8.3 | 0.001 | 59.2±10.4 | 0.006 | 21.1±9.9 | 0.23 |
| 0.1 | 98.9±20.4 | 72.5±12.6 | 0.002 | 61.6±13.6 | 0.006 | 22.2±8.3 | 0.10 |
| 1 | 99.1±22.3 | 73.1±11.7 | 0.001 | 68.8±17.1 | 0.0008 | 22.4±8.0 | 0.075 |
| 10 | 102.4±24.5 | 69.6±10.5 | 0.004 | 68.4±13.4 | 0.0001 | 22.6±9.5 | 0.10 |
| 100 | 99.2±30.7 | 31.7±8.8 | | 7.2±4.5 | | -1.3±1.3 | |

### [Results]

The data listed in Table 1 and shown in Fig. 1 indicate that diclofenac sodium (DFNa) inhibits the growth of the corneal epithelial cells at a concentration of 0.25mM, 0.5mM or 1mM in a dose-dependent manner. The concentration corresponds to 10 times the foregoing concentration thereof required for inhibiting any growth of the epithelial cells of the lens. It was found that the addition of MS in an amount ranging from 0.01 to 10 µ M could significantly promote the cell-proliferation. MS per se had a cytotoxic effect at a concentration of 100 µ M.

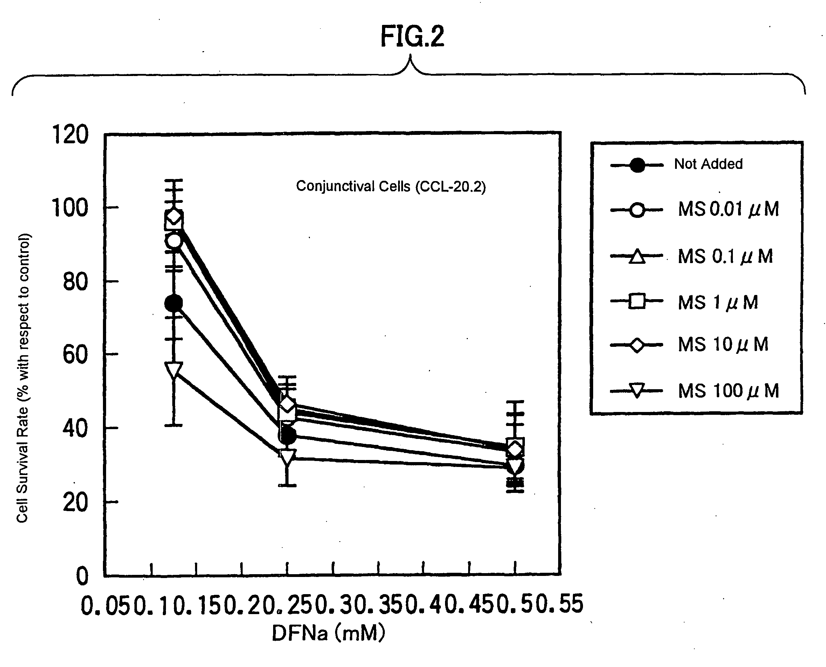

Table 2 and Fig. 2 show the protective effect of misoprostol on corneal disorders of human conjunctival epithelial cells caused by the action of non-steroidal anti-inflammatory agent.

**Table 2:**

| Effect of Misoprostol on Disorders of Conjunctival Epithelial Cells Caused by Diclofenac Sodium | | | | | | | |
|---|---|---|---|---|---|---|---|
| Concn. of MS (µ M) | Concentration of Diclofenac Sodium (mM) | | | | | | |
| | 0 | 0.125 | p Value | 0.25 | p Value | 0.5 | p Value |
| 0 | 100(%) | 74.0±10.0 | | 37.8±5.7 | | 29.3±5.6 | |
| 0.01 | 93.0±14.8 | 91.1±8.1 | 0.0001 | 42.6±4.9 | 0.037 | 33.1±7.4 | 0.17 |
| 0.1 | 98.6±20.2 | 97.1±4.5 | 3 X 10⁻⁷ | 45.0±6.7 | 0.029 | 34.2±9.3 | 0.13 |
| 1 | 104.0±26.6 | 96.4±8.4 | 6 X 10⁻⁶ | 44.0±6.4 | 0.019 | 34.4±12.2 | 0.20 |
| 10 | 99.5±22.2 | 97.8±9.6 | 6 X 10⁻⁶ | 46.4±7.3 | 0.004 | 33.7±9.4 | 0.18 |
| 100 | 86.3±12.0 | 55.4±14.7 | | 31.5±7.4 | | 28.7±6.4 | |

### [Results]

The data listed in Table 2 and shown in Fig. 2 indicate that diclofenac sodium inhibits the growth of the conjunctival epithelial cells at a concentration of 0.125mM, 0.25mM or 0.5mM in a dose-dependent manner. It was recognized that MS could eliminate the cell proliferation-inhibitory action at a concentration of 0.1, 1 and 10 µ M. The effect of MS was found to be reduced at a high concentration of diclofenac sodium. MS per se had a cytotoxic effect at a concentration of 100 µ M.

### Industrial Applicability

The present invention relates to an eye drop for treating or preventing disorders of corneal and/or conjunctival epithelial cells, which comprises prostaglandin E's such as misoprostol and the eye drop shows a high effect of treating disorders of cornea or conjunctiva originated from physical, chemical and/or mechanical damages of the cornea or conjunctiva as well as endogenous disorders of cornea or conjunctiva. In particular, the eye drop of the present invention shows an effect of treating disorders of cornea due to a non-steroidal anti-inflammatory agent such as diclofenac sodium or controlling the cell proliferation-inhibitory action of such an agent.

## Claims

1. An eye drop for treating or preventing disorders of corneal and/or conjunctival epithelial cells comprising prostaglandin E's as effective components.

2. The eye drop as set forth in claim 1, wherein the prostaglandin E's are selected from the group consisting of misoprostol, prostaglandin E1 (PGE1), prostaglandin E2 (PGE2), 15R-methyl prostaglandin E2 (15R-methyl PGE2), 16,16-dimethyl prostaglandin E1 (16,16-dimethyl PGE1), 16,16-dimethyl prostaglandin E2 (16,16-dimethyl PGE2), oxyprostol and lower alkyl esters thereof.

3. The eye drop as set forth in claim 1 or 2, wherein the disorders of corneal and/or conjunctival epithelial cells are caused due to the reduction of prostaglandin E's.

4. The eye drop as set forth in claim 1 or 2, wherein the disorders of corneal and/or conjunctival epithelial cells are caused due to the use of a drug.

5. The eye drop as set forth in claim 4, wherein the causative drug for the disorders of corneal and/or conjunctival epithelial cells is an anti-inflammatory agent.

6. The eye drop as set forth in claim 5, wherein the anti-inflammatory agent as a causative drug for the disorders of corneal and/or conjunctival epithelial cells is a drug non-selectively inhibiting cyclooxygenase.

7. The eye drop as set forth in claim 6, wherein the drug non-selectively inhibiting cyclooxygenase as a causative drug for the disorders of corneal and/or conjunctival epithelial cells is diclofenac sodium.

8. An eye drop for treating or preventing disorders of corneal and/or conjunctival epithelial cells comprising prostaglandin E's and a causative drug for the disorders of corneal and/or conjunctival epithelial cells, as effective components.

9. The eye drop as set forth in claim 8, wherein the causative drug for the disorders of corneal and/or conjunctival epithelial cells is an anti-inflammatory agent.

10. The eye drop as set forth in claim 9, wherein the ratio, by mass, of the prostaglandin E's to the anti-inflammatory agent ranges from 1:25 to 50000.

11. The eye drop as set forth in claim 10, wherein the prostaglandin E is misoprostol and the anti-inflammatory agent is diclofenac sodium.

12. The eye drop as set forth in claim 11, wherein the concentration of the misoprostol ranges from 0.01 µ M to 10 µ M.

13. The eye drop as set forth in claim 11, wherein the concentration of the diclofenac sodium ranges from 0.01% to 0.5%.
